# EUROPEAN PATENT APPLICATION

(11) **EP 2 502 506 A1**
(43) Date of publication of application: **26.09.2012**
(21) Application number: 11380021.3
(22) Date of filing: 21.03.2011
(51) Int. Cl.: A23L 1/30, A61K 31/201, A61K 31/202, A61P 19/00

(54) **Methods for improving bone health in infants using long chain polyunsaturated fatty acids**

(71) Applicant: Abbott Laboratories, Abbott Park, IL 60064 (US)
(72) Inventor: Lopez Pedrosa, José Maria, 18009 Granada (ES); Manzano Martin, Manuel, 18151 Granada (ES); Rueda Cabrera, Ricardo, 18008 Granada (ES)
(74) Representative: Ungria Lopez, Javier

(57) **Abstract**

Disclosed are methods for improving bone health, strength and formation in an infant who may be susceptible to developing bone health issues from conception through adolescence. The methods include administration of a nutritional formulation including at least one LCPUFA to a woman during pregnancy and optionally during lactation and breastfeeding of an infant.

## Description

### FIELD OF THE DISCLOSURE

The present disclosure relates to methods of improving bone health, bone strength, and bone formation of an infant by administration of long chain polyunsaturated fatty acids to the mother of the infant during pregnancy and optionally during breastfeeding and after weaning. The methods of the present disclosure improve bone health, bone strength, and bone formation in the infant from conception through adolescence.

### BACKGROUND OF THE DISCLOSURE

The skeleton is a vital organ that undergoes substantial changes in shape and structure throughout growth and is exposed to continual processes of removal and renewal throughout life. The bones of children need to be of the size and shape appropriate for their ages, and of sufficient strength to support current activity. In addition to these immediate needs, however, at the completion of growth, the bones must be able to meet the lifetime load-bearing demands of adulthood. Excessive fragility of the aging skeleton is due in large part to inadequate increases in apparent bone density and, at least in the spine, failure to attain sufficient size. The bone mass accrual and bone formation occurring during childhood and adolescence is a determinant of peak bone mass. Accordingly, maximizing bone mass in these periods may be an important factor for preventing osteoporosis, and associated fracture later in life.

Some infants are born with under-developed skeletons such that their bone health, bone strength, and bone formation is compromised. As these infants grow into children and adolescents, their overall bone health and strength may limit their activity as well as impact their lives as they become adults and the natural aging process occurs. When born with an under-developed skeletal system, it may be difficult in some cases for the infant to overcome the related challenges throughout life. Additionally, under-developed skeletons may also increase the likelihood of adult bone issues including, for example, osteoporosis.

Accordingly, there is a continuing need for nutritional means of effectively improving bone health, bone strength, and bone formation in an infant from conception through adolescence. It would be beneficial if the means included components that could be consistently administered to the pregnant mother from conception through delivery and breastfeeding of the infant, and possibly to the infant after weaning.

### SUMMARY OF THE DISCLOSURE

One embodiment of the present disclosure is directed to a method of improving bone health in an infant susceptible to developing bone health issues. The method comprises administering to a pregnant woman a nutritional formulation comprising at least one long chain polyunsaturated fatty acid.

Another embodiment is directed to a method of increasing bone strength in an infant susceptible to developing bone health issues. The method comprises administering to a pregnant woman a nutritional formulation comprising at least one long chain polyunsaturated fatty acid.

Another embodiment is directed to a method of promoting bone formation in an infant susceptible to developing bone health issues. The method comprises administering to a pregnant woman a nutritional formulation comprising at least one long chain polyunsaturated fatty acid and administering to the woman the nutritional formulation following delivery of the infant and during breastfeeding of the infant. The nutritional formulation comprises an amount of long chain polyunsaturated fatty acid effective to promote bone formation in the infant.

It has been discovered that fortifying the maternal diet during pregnancy and optionally during lactation and breastfeeding with long chain polyunsaturated fatty acids (LCPUFAs) can result in improved bone health, bone strength, and bone formation in an infant, from conception through adolescence. Particularly, the LCPUFAs, and specifically n-3 LCPUFAs, have been shown to modulate bone marrow mesenchymal stem cell (MSC) differentiation and to induce mature mineralizing osteoblastic phenotype during the early stage of bone development, which are determinants of bone health, bone strength, and bone formation.

By supplementing the pregnant and optionally the lactating woman's diet with effective amounts of LCPUFAs, peak bone mass and bone mass density of the infant are increased, providing the infant with adequate skeletal mass and strength required throughout adolescence and adulthood. These effects may further prevent osteoporosis and associated fractures later in life. These benefits may be furthered by supplementing the infant's diet after weaning with LCPUFAs.

### BRIEF DESCRIPTION OF THE FIGURES

Figure 1 depicts the effects of maternal nutritional intervention on whole body bone mass density (BMD) and bone mineral content (BMC) of rat offspring at weaning as analyzed in Example 1.
Figure 2 depicts the effects of maternal nutritional intervention on vertebra trabecular architecture of rat offspring at weaning as analyzed in Example 1.
Figure 3 depicts the effects of maternal nutritional intervention on whole body bone mass density (BMD) and bone mineral content (BMC) of rat offspring at the adolescent period as analyzed in Example 1.
Figure 4 depicts the effects of maternal nutritional intervention on vertebra trabecular architecture of rat offspring at the adolescent period as analyzed in Example 1.
Figure 5 depicts the effects of maternal nutritional intervention on vertebra cortical architecture of rat offspring at the adolescent period as analyzed in Example 1.
Figures 6A and 6B depict the expression of osteocalcin (OCN), alkaline phosphatase (ALP) and Type 1 Collagen by human bone marrow cells grown in media supplemented with docosahexaenoic acid (DHA) as compared to those of the basal control cells as analyzed in Example 2.
Figures 6C and 6D depict the expression of OCN, ALP and Type 1 Collagen by human bone marrow cells grown in media supplemented with eicosapentaenoic acid (EPA) as compared to those of the basal control cells as analyzed in Example 2.

### DETAILED DESCRIPTION OF THE DISCLOSURE

The nutritional formulations used in the methods of the present disclosure comprise at least one LCPUFA to improve bone health, bone strength, bone, and bone formation (including bone mass accretion and architecture) in an infant from conception of the infant through adolescence. The essential features of the methods of the present disclosure and the nutritional formulations used therein, as well as some of the many optional variations and additions, are described in detail hereafter.

The terms "nutritional formulation" or "nutritional composition" as used herein, are used interchangeably and, unless otherwise specified, refer to nutritional liquids, nutritional powders, nutritional bars, nutritional supplements, and any other nutritional food product as known in the art. The nutritional powders may be reconstituted to form a nutritional liquid. The nutritional formulation or nutritional composition may include at least one of fat, protein and carbohydrate, and are suitable for oral consumption by a human.

The term "nutritional liquid" as used herein, unless otherwise specified, refers to nutritional products in ready-to-drink liquid form, concentrated form, and nutritional liquids made by reconstituting the nutritional powders described herein prior to use.

The term "nutritional powder" as used herein, unless otherwise specified, refers to nutritional formulations in flowable or scoopable form that can be reconstituted with water or another aqueous liquid prior to consumption and includes both spray dried and dry mixed/dry blended powders.

The term "infant" as used herein, unless otherwise specified, refers to a child 12 months or younger.

The term "adolescence" as used herein, unless otherwise specified, refers to the stage of life ending at age 12 years.

The term "infant formula" as used herein, unless otherwise specified, refers to liquid and solid nutritional formulations suitable for consumption by an infant.

The term "follow-on formula" as used herein, unless otherwise specified, refers to liquid and solid nutritional formulations suitable for consumption by a child older than 12 months up to 36 months.

The term "pediatric formula" as used herein, unless otherwise specified, refers to liquid and solid nutritional formulations suitable for consumption by a child older than 36 months up to age 12 years.

All percentages, parts and ratios as used herein, are by weight of the total product, unless otherwise specified. All such weights as they pertain to listed ingredients are based on the active level and, therefore, do not include solvents or by-products that may be included in commercially available materials, unless otherwise specified.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The various embodiments of the nutritional formulations used in the methods of the present disclosure may also be substantially free of any optional or selected essential ingredient or feature described herein, provided that the remaining formulation still contains all of the required ingredients or features as described herein. In this context, and unless otherwise specified, the term "substantially free" means that the selected formulation contains less than a functional amount of the optional ingredient, typically less than about 1%, including less than about 0.5%, including less than about 0.1%, and also including zero percent, by weight of such optional or selected essential ingredient.

The nutritional formulations and methods may comprise, consist of, or consist essentially of the essential elements of the products as described herein, as well as any additional or optional element described herein or otherwise useful in nutritional formulation applications.

### Product Form

The nutritional formulations used in the methods of the present disclosure may be formulated and administered in any known or otherwise suitable oral product form. Any solid, liquid, or powder form, including combinations or variations thereof, are suitable for use herein, provided that such forms allow for safe and effective oral delivery to the individual of the essential ingredients as also defined herein. The nutritional formulations may be formulated to include only the essential ingredients described herein, or may be modified with optional ingredients to form a number of different product forms.

The nutritional formulations used in the methods of the present disclosure may be formulated to comprise at least one of fat, protein, and carbohydrate, and preferably also contain vitamins, minerals, or combinations thereof. The formulation also comprises at least one LCPUFA.

The nutritional formulations may be formulated with sufficient kinds and amounts of nutrients to provide a sole, primary, or supplemental source of nutrition, or to provide a specialized nutritional formulation.

Specific non-limiting examples of product forms include nutritional formulations for pregnant and lactating women and infant formulas, including both preterm and term infant formulas. Other examples of product forms include human milk fortifiers, follow-on formulas, pediatric formulas, and adult nutritional formulations.

### Nutritional Solids

The nutritional solids may be in any form, including nutritional bars, nutritional tablets, and the like, but are typically in the form of flowable or substantially flowable particulate formulations, or at least particulate formulations. Particularly suitable nutritional solid product forms include spray dried, agglomerated or dryblended powder compositions. The formulations can easily be scooped and measured with a spoon or similar other device, wherein the formulations can easily be reconstituted by the intended user with a suitable aqueous liquid, typically water, to form a nutritional formulation for immediate oral or enteral use. In this context, "immediate" use generally means within about 48 hours, most typically within about 24 hours, preferably right after reconstitution.

### Nutritional Liquids

Nutritional liquids include both concentrated and ready-to-feed nutritional liquids. These nutritional liquids are most typically formulated as suspensions, emulsions or clear or substantially clear liquids.

Nutritional emulsions suitable for use may be aqueous emulsions comprising proteins, fats, and carbohydrates. These emulsions are generally flowable or drinkable liquids at from about 1 °C to about 25°C and are typically in the form of oil-in-water, water-in-oil, or complex aqueous emulsions, although such emulsions are most typically in the form of oil-in-water emulsions having a continuous aqueous phase and a discontinuous oil phase.

The nutritional emulsions may be and typically are shelf stable. The nutritional emulsions typically contain up to about 95% by weight of water, including from about 50% to about 95%, also including from about 60% to about 90%, and also including from about 70% to about 85%, of water by weight of the nutritional emulsions. The nutritional emulsions may have a variety of product densities, but most typically have a density greater than about 1.03 g/ml, including greater than about 1.04 g/ml, including greater than about 1.055 g/ml, including from about 1.06 g/ml to about 1.12 g/ml, and also including from about 1.085 g/ml to about 1.10 g/ml.

The nutritional emulsion may have a pH ranging from about 3.5 to about 8, but are most advantageously in a range of from about 4.5 to about 7.5, including from about 5.5 to about 7.3, including from about 6.2 to about 7.

### Long Chain Polyunsaturated Fatty Acids (LCPUFAs)

The nutritional formulations used in the methods of the present disclosure include at least one LCPUFA. LCPUFAs are included in the nutritional formulation to at least modulate bone marrow mesenchymal stem cell (MSC) differentiation and to induce mature mineralizing osteoblastic phenotype during the early stage of bone development to provide improved bone health, strength, and formation.

Exemplary LCPUFAs for use in the nutritional formulations include, for example, n-3 LCPUFAs and n-6 LCPUFAs. Specific LCPUFAs include docosahexaenoic acid (DHA), eicosapentaenoic acid (EPA), arachidonic acid (ARA), linoleic acid, linolenic acid (alpha linolenic acid) and gamma-linolenic acid derived from oil sources such as plant oils, marine plankton, fungal oils, and fish oils. In one particular embodiment, the LCPUFAs are derived from fish oils such as menhaden, salmon, anchovy, cod, halibut, tuna, or herring oil. Particularly preferred LCPUFAs include DHA and EPA, and particularly a combination of DHA and EPA. One particularly preferred LCPUFA source is Eupoly-DHA® (Puleva Biotech©, Spain), which is a refined marine oil including 10% EPA and 18% DHA.

The nutritional formulations as described herein will typically comprise from about 0.001% to about 1.0%, including from about 0.001% to about 0.1 %, including from about 0.02% to about 0.09%, including from about 0.025% to about 0.06%, including from about 0.025% to about 0.05% LCPUFA by weight of the nutritional formulation.

For administration to pregnant women or lactating and breastfeeding women, the nutritional formulations generally provide total LCPUFAs in an amount of from about 100 mg/day to 2000 mg/day, including from about 200 mg/day to about 2000 mg/day, including from about 500 mg/day to about 1500 mg/day, including from about 1000 mg/day to about 1400 mg/day.

In one particularly preferred embodiment, the nutritional formulations provide from about 200 mg/day to about 1000 mg/day of DHA and from about 100 mg/day to about 500 mg/day of EPA to the pregnant and breastfeeding woman. In addition, in embodiments where the nutritional formulation comprises both EPA and DHA, the weight ratio of EPA to DHA is from about 1:2 to about 1:4, including from about 1:2 to about 1:3, including about 1:2.

In another embodiment, the amount of LCPUFAs provided by the nutritional formulation will depend on the pregnant woman's physical need which increases throughout the pregnancy such that the need for LCPUFAs later in pregnancy is greater than in the beginning. For example, when DHA is administered to a pregnant woman, the amount of DHA provided by the nutritional formulation varies according to the stage of pregnancy.

In a further embodiment, when the nutritional formulation is provided to an infant, the nutritional formulation comprises from about 0.001% to about 1.0%, including from about 0.001% to about 0.1 %, including from about 0.02% to about 0.09%, including from about 0.025% to about 0.06%, including from about 0.025% to about 0.05% LCPUFA by weight of the nutritional formulation. Similar amounts may be suitable for use through adolescence.

### Macronutrients

The nutritional formulation may further comprise one or more optional macronutrients in addition to the LCPUFA described herein. The optional macronutrients include proteins, lipids, carbohydrates, and combinations thereof. The nutritional compositions are desirably formulated as nutritional formulations containing all three macronutrients.

Macronutrients suitable for use herein include any protein, lipid, or carbohydrate or source thereof that is known for or otherwise suitable for use in an oral nutritional formulation, provided that the optional macronutrient is safe and effective for oral administration and is otherwise compatible with the other ingredients in the nutritional formulation.

The concentration or amount of optional lipid, carbohydrate, and protein in the nutritional formulation can vary considerably depending upon the particular product form (e.g., bars or other solid dosage forms, milk or soy-based liquids/emulsions or other clear beverages, reconstitutable powders etc.) and the various other formulations and targeted dietary needs. These optional macronutrients are most typically formulated within any of the embodied ranges described in the following tables.

**Each numerical value preceded by the term "about"**

| Nutrient (% total calories) | Example A | Example B | Example C |
|---|---|---|---|
| Carbohydrate | 0-100 | 10-70 | 40-50 |
| Lipid | 0-100 | 20-65 | 35-55 |
| Protein | 0-100 | 5-40 | 15-25 |

**Each numerical value preceded by the term "about"**

| Nutrient (wt% composition) | Example D | Example E | Example F |
|---|---|---|---|
| Carbohydrate | 0-98 | 1-50 | 10-30 |
| Lipid | 0-98 | 1-30 | 3-15 |
| Protein | 0-98 | 1-30 | 2-10 |

### Carbohydrate

Optional carbohydrates suitable for use in the nutritional formulations may be simple, complex, or variations or combinations thereof, all of which are optionally in addition to the LCPUFAs as described herein. Non-limiting examples of suitable carbohydrates include hydrolyzed or modified or resistant starch or cornstarch, maltodextrin, isomaltulose, sucromalt, glucose polymers, sucrose, corn syrup, corn syrup solids, rice-derived carbohydrate, glucose, fructose, lactose, high fructose corn syrup, honey, sugar alcohols (e.g., maltitol, erythritol, sorbitol), and combinations thereof.

Optional carbohydrates suitable for use herein also include soluble dietary fiber, non-limiting examples of which include gum Arabic, sodium carboxymethyl cellulose, guar gum, citrus pectin, low and high methoxy pectin, oat and barley glucans, carrageenan, psyllium and combinations thereof. Insoluble dietary fiber is also suitable as a carbohydrate source herein, non-limiting examples of which include oat hull fiber, pea hull fiber, soy hull fiber, soy cotyledon fiber, sugar beet fiber, cellulose, corn bran, and combinations thereof.

### Protein

Optional proteins suitable for use in the nutritional formulations include hydrolyzed, partially hydrolyzed or non-hydrolyzed proteins or protein sources, and can be derived from any known or otherwise suitable source such as milk (e.g., casein, whey), animal (e.g., meat, fish, egg albumen), cereal (e.g., rice, corn), vegetable (e.g., soy, pea, potato), or combinations thereof. The proteins for use herein can also include, or be entirely or partially replaced by, free amino acids known for use in nutritional products, non-limiting examples of which include L-tryptophan, L-glutamine, L-tyrosine, L-methionine, L-cysteine, taurine, L-arginine, L-camitine, and combinations thereof.

### Lipid

Optional lipids suitable for use in the nutritional formulations include coconut oil, fractionated coconut oil, soy oil, corn oil, olive oil, safflower oil, high oleic safflower oil, high GLA-safflower oil, MCT oil (medium chain triglycerides), sunflower oil, high oleic sunflower oil, palm and palm kernel oils, palm olein, canola oil, flaxseed oil, borage oil, cottonseed oils, evening primrose oil, blackcurrant seed oil, transgenic oil sources, fungal oils, marine oils (e.g., tuna, sardine) and so forth.

### Optional Ingredients

The nutritional formulations may further comprise other optional ingredients that may modify the physical, nutritional, chemical, hedonic or processing characteristics of the products or serve as pharmaceutical or additional nutritional components when used in a targeted population. Many such optional ingredients are known or otherwise suitable for use in other nutritional products and may also be used in the nutritional formulations described herein, provided that such optional ingredients are safe and effective for oral administration and are compatible with the essential and other ingredients in the formulation.

Non-limiting examples of such other optional ingredients include preservatives, anti-oxidants, buffers, pharmaceutical actives, sweeteners, colorants, flavors, flavor enhancers, thickening agents and stabilizers, emulsifying agents, prebiotics, lubricants, and combinations thereof.

The nutritional formulations may further include one or more minerals, non-limiting examples of which include phosphorus, sodium, chloride, magnesium, manganese, iron, copper, zinc, iodine, calcium, potassium, chromium, molybdenum, selenium, and combinations thereof.

The nutritional formulations may also include one or more vitamins, non-limiting examples of which include carotenoids (e.g., beta-carotene, zeaxanthin, lutein, lycopene), biotin, choline, inositol, folic acid, pantothenic acid, choline, vitamin A, thiamine (vitamin B1), riboflavin (vitamin B2) niacin (vitamin B3), pyridoxine (vitamin B6), cyanocobalamin (vitamin B12), ascorbic acid (vitamin C), vitamin D, vitamin E, vitamin K, and various salts, esters, or other derivatives thereof, and combinations thereof.

### Methods of Manufacture

The nutritional liquids may be manufactured by any known or otherwise suitable method for making nutritional liquids, including emulsions such as milk-based nutritional emulsions.

In one suitable manufacturing process, a nutritional liquid is prepared using at least three separate slurries, including a protein-in-fat (PIF) slurry, a carbohydrate-mineral (CHO-MIN) slurry, and a protein-in-water (PIW) slurry. The PIF slurry is formed by heating and mixing the selected oils (e.g., canola oil, corn oil, fish oil (including LCPUFAs), etc.) and then adding an emulsifier (e.g., lecithin), fat soluble vitamins, and a portion of the total protein (e.g., milk protein concentrate, etc.) with continued heat and agitation. The CHO-MIN slurry is formed by adding with heated agitation to water: minerals (e.g., potassium citrate, dipotassium phosphate, sodium citrate, etc.), trace and ultra trace minerals (TM/UTM premix), thickening or suspending agents (e.g. Avicel, gellan, carrageenan). The resulting CHO-MIN slurry is held for 10 minutes with continued heat and agitation before adding additional minerals (e.g., potassium chloride, magnesium carbonate, potassium iodide, etc.) and/or carbohydrates (e.g., sucrose, corn syrup, etc.). The PIW slurry is then formed by mixing with heat and agitation the remaining protein (e.g., sodium caseinate, soy protein concentrate, etc.) into water.

The resulting slurries are then blended together with heated agitation and the pH adjusted to the desired range, typically from 6.6-7.0, after which the composition is subjected to high-temperature short-time (HTST) processing during which the composition is heat treated, emulsified and homogenized, and then allowed to cool. Water soluble vitamins and ascorbic acid are added, the pH is again adjusted to the desired range if necessary, flavors are added, and water is added to achieve the desired total solid level. The composition is then aseptically packaged to form an aseptically packaged nutritional emulsion, or the composition is added to retort stable containers and then subjected to retort sterilization to form retort sterilized nutritional emulsions.

The manufacturing processes for the nutritional emulsions may be carried out in ways other than those set forth herein without departing from the spirit and scope of the present disclosure. The present embodiments are, therefore, to be considered in all respects illustrative and not restrictive and that all changes and equivalents also come within the description of the present disclosure.

The nutritional solid, such as a spray dried nutritional powder or dry-mixed nutritional powder, may be prepared by any collection of known or otherwise effective techniques, suitable for making and formulating a nutritional powder.

For example, when the nutritional powder is a spray-dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid and then spray drying the slurry or liquid to produce a spray dried nutritional powder. The method may further comprise the step of spray drying, dry mixing, or otherwise adding additional nutritional ingredients, including any one or more of the ingredients described herein, to the spray dried nutritional powder.

### Methods of Use

The nutritional formulations used in the methods of the present disclosure are administered to a pregnant woman and through delivery of the infant and optionally subsequently to the woman during lactation and breastfeeding and further optionally to the infant after weaning to provide the infant with LCPUFAs prior to delivery and optionally after delivery. The pregnant or lactating woman receiving the nutritional formulation may be a woman with no family history of bone health problems, including fragile bones or osteoporosis, or may be a woman with a family history of bone health problems, including fragile bones or osteoporosis such that any offspring of the woman may be susceptible to developing bone health issues and/or may be at risk of having or developing bone health problems, such as osteoporosis, fragile bones, and the like. The methods of the present disclosure may be particularly suited for infants who are at risk of developing bone health issues, such as but not limited to osteoporosis, due to family history.

In some embodiments, the nutritional formulations comprising the LCPUFAs are additionally administered to the woman following delivery and during lactation and breastfeeding of the infant such that the infant continues to realize the bone benefits provided by the LCPUFAs after delivery. The nutritional formulations may be administered to the woman orally as needed to provide the desired level of nutrition, although it is generally desirable that the administration of the nutritional formulation comprising the LCPUFAs be administered at least once daily, including twice daily, including three times daily or more during pregnancy and lactation and breastfeeding of the infant.

In some embodiments, the nutritional formulations comprising the LCPUFAs may also be administered directly to the infant after weaning to further provide LCPUFAs to the growing and developing infant. Typically, in this embodiment, the nutritional formulations may be administered from weaning through adolescence and any time period included therein at least once daily, including twice daily, including three times daily or more. After weaning, the nutritional formulation provided to the infant and including the LCPUFAs may be in the form of an infant formula, and potentially, a follow-on formula, a pediatric formula, and the like, depending upon the age of the infant. In accordance with some embodiments of the present disclosure, the infant may continue to receive the nutritional formulation after weaning through adolescence.

The use of LCPUFAs in the nutritional formulations used in the methods of the present disclosure provides improved bone health, improved bone strength, improved bone mass accretion and architecture and bone formation in an infant from conception through adolescence, including from conception through infancy. Further, the use of LCPUFAs in the nutritional formulations used in the methods of the present disclosure modulate bone marrow mesenchymal stem cell (MSC) differentiation and induce mature mineralizing osteoblastic phenotype during the early stage of bone development in an infant. In addition, the use of LCPUFAs in the nutritional formulations used in the methods of the present disclosure improves absorption of bone minerals and improves bone mineral density in the infant.

### EXAMPLES

The following examples illustrate specific embodiments and/or features of the methods of the present disclosure. The examples are given solely for the purpose of illustration and are not to be construed as limitations of the present disclosure, as many variations thereof are possible without departing from the spirit and scope of the disclosure. All exemplified amounts are weight percentages based upon the total weight of the product, unless otherwise specified.

The exemplified products are nutritional formulations prepared in accordance with manufacturing methods well known in the nutrition industry for preparing nutritional emulsions and powders.

### Example 1

In this Example, the effect of maternal diet supplementation with (1) Prebiotics; or (2) LCPUFAs; or (3) Calcium; or (4) Vitamin D on fetal and postnatal skeletal development is analyzed.

Fifty 10-week-old pregnant Sprague-Dawley rats at the eleventh day of gestation (Charles Rivers Laboratories, Orleans Cedex, France) are housed under standardized environmental conditions (22°C, relative humidity of 50%, artificial lighting on for 12 hours/day) and are given free access to deionized water during the test period. The rats are randomly divided into five groups of feeding (n=10/group): (1) Control Group ("Control"), who receives a standard purified rodent diet (SPRD); (2) Calcium group ("Calcium"), who receives the SPRD fortified with 0.5% calcium carbonate (final concentration in the diet 1.0 g Ca²⁺ per 100g product); (3) Prebiotic group ("Prebiotic"), who receives the SPRD containing 7.5% of the total carbohydrate as inulin-type fructans (Synergy-1®, Orafti, Belgium); (4) LCPUFA group ("LCPUFA"), who receives the SPRD containing 50% of the total fat in the form of n-3 long chain polyunsaturated fatty acids (Eupoly-DHA®, 10% EPA and 18% DHA, Puleva Biotech, Spain); and (5) Vitamin D group ("Vitamin D"), who receives the SPRD fortified with a final concentration of 1200 IU of Vitamin D per 100g of product (Vitamin D, DSM Nutritional Products Europe LTD, Spain).

After delivery, pups from the same group are mixed and 8 pups, 5 females and 3 males, are randomly housed to one dam. During their suckling period, the pups receive only the dam's milk. The Dam's nutritional treatment is finished at weaning (pups, 25-day-old) and the weaning pups are fed the SPRD during the growing period until adolescence (offspring, 90-day-old).

Sacrifices of rat pups are carried out at birth, weaning and at the end of the adolescence period. The whole bone mineral density (BMD) and bone mineral content (BMC) of the skeleton are measured at birth and at weaning by using a pDEXA densitometer (Norland Corp., Fort Atkinson, WI, USA). In adolescent offspring, the determination of BMC and BMD are analyzed in isolated bones (femur, tibia and vertebrae). Determination of the trabecular architecture parameters (trabecular thickness, separation and number), bone length and bone volume fraction (BV/TV) is analyzed by Micro-computed tomography (micro-CT). Micro-CT analysis is carried out by the Bone & Joint Research Group (University of Southampton, UK) and cortical parameters are performed by SCANCO Medical AG (Brüttisellen, Switzerland).

### Dual energy X-ray absorptiometry (DEXA)

DEXA scan is a technique used in the clinical practice for the analysis of bone health. Two main bone properties can be measured by this technique: Bone Mineral Content (BMC) and areal Bone Mineral Density (BMD). BMC determines the mass of mineral present in the whole body or in the selected skeletal region. BMC changes reflect the result of the metabolic "mass" balance between bone formation and bone destruction. BMC represented relative to the projected bone area refers to BMD. BMD represents the whole mass of mineral present in the bone region studied.

In this Example, the measurements of BMC (in grams) and BMD (in g/cm²) are assessed by pDEXA densitometer and are performed by the same technician. The manufacturer's software for research in small animals calculates BMC and BMD for the isolated bone. Femur is measured from the femoral neck to the knee joint. Tibia is measured from the knee joint to the joint between tibia and calcaneus, also including fibula. Vertebra is measured from the inferior level of lumbar vertebra 3 to the superior level of lumbar vertebra 5 (LV3-LV5).

### Micro-CT

Micro-computed tomography (micro-CT) is a technique for the nondestructive assessment and analysis of the three-dimensional trabecular and cortical bone structural properties.

Scanning with micro-CT can be achieved at resolutions as low as 5 *µ*m, allowing for the determination of porosities and subtle modeling and remodeling events of the bone tissue. Furthermore, true three-dimensional image reconstructions permit the assessment of bone microarchitecture as a three dimensional structure, providing critical information to images collected through histomorphometry.

In the present Example, femoral, tibia and vertebra cancellous bones are assessed using Metris (XTek) Benchtop 160Xi CT scanner (University of Southampton) and vertebra cortical bone is assessed using MicroCT-40 computed tomography system (Scanco Medical, Basserdorf, Switzerland). Micro-CT imaging is coupled with stereological methods to estimate bone volume fraction (BV/TV, is a 3-D parameter that relates trabecular bone tissue per unit of total volume (mineralised and nonmineralised tissue) and is one of the most important parameters to characterize cancellous bone architectural morphology) and 3-D parameters of trabecular architecture including: trabecular number (Tb.N), trabecular thickness (Tb.Th in *µ*m, average thickness of trabeculae), and trabecular separation (Tb.Sp in *µ*m, the average distance between trabeculae, representing the amount of marrow space).

Micro-CT analysis also allows for the determination of various quality properties of the cortical bone. The bone fragility is influenced not only by the architecture but also by the tissue "quality". The strength of a bone as an organ depends on the material properties of bone as a tissue. Stiffness, or the resistance of the whole bone to deformation by a load, is a key material property and requires a destructive mechanical technique if measurements are done *in vivo.* The nondestructive analysis of the volumetric bone mineral density of cortical bone (cortical vBMD) by micro CT has an approximately linear relationship with the stiffness of the cortical bone. Cortical thickness (C.Th) is one of the variables that helps to evaluate the modeling-derived changes provoked by growth. The cortical porosity (C.Sp) reflects the intracortical remodeling in the bone. Remodeling, that occurs in the surfaces of bone, could be enhanced if the pore size becomes larger and, therefore, with the increase of the cortical porosity. An increment on cortical porosity weakens the bone and decreases the bone strength.

### Statistical analysis

Results are expressed as mean ± SEM, and the probability level at which the differences are considered significant is set at p < 0.05. One-way ANOVA is used to evaluate differences attributable to the diet in the different markers, previously testing the homogeneity of variances by Levene's. A posteriori post hoc tests are carried out by the Dunnet test.

### Results

### Delivery

At delivery, only the pups whose mothers received the Calcium fortified diet have a significantly lower weight in comparison to Control group (Calcium group: 8.24 ± 0.10 g; Control group: 8.65 ± 0.12 g; p<0.05 vs. Control group). This group also shows significant differences respective to the Control group on the trabecular parameters of the appendicular bones, femur and tibia, having lower trabecular thickness but greater trabecular number. This implicates a decrease in the trabecular separation, although statistical differences are not achieved. The differences found on the trabecular parameters are mainly due to the smaller size of the litter in the Calcium group. In fact, no difference respective to Control group is observed when femur length is represented versus body weight, showing that the Calcium group has a normal constitution (Calcium group: 0.572 ± 0.04; Control group: 0.552 ± 0.05).

No differences between groups are observed on the trabecular parameters of the lumbar vertebra at this period.

### Weaning

At weaning period, the pups whose mothers received the Calcium-fortified diet or Prebiotic supplemented diet show a statistical lower body weight as compared with Control group (Calcium group: 62.0 ± 0.9 g; Prebiotic group: 67.6 ± 1.0 g; Control group: 72.2 ± 1.3 g; p<0.05 vs Control group). In contrast, pups whose mothers received the LCPUFAs-supplemented diet show a higher body weight (LCPUFA group: 76.8 ± 1.4 g; Control group: 72.2 ± 1.3 g; p<0.05 vs. Control group).

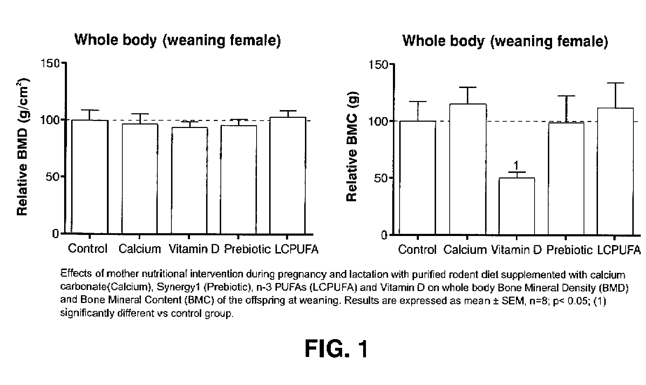

Whole body BMD and BMC of the pups at this period are shown in FIG. 1. Only the pups whose mother received a Vitamin D-fortified diet show a lower whole body BMC relative to Control group.

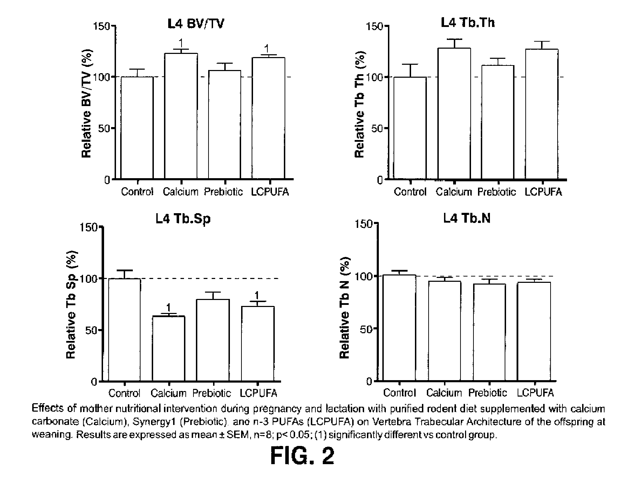

A beneficial effect due to nutritional intervention is found in the vertebra trabecular architecture. Trabecular separation and BV/TV ratio is statistically different for Calcium and LCPUFA groups respective to Control group. Although statistically significant differences are not achieved, pups whose mother received Prebiotics show a 7% higher BV/TV of the vertebra than the pups of Control group. In addition, about a 30% increase respective to Control group is achieved in the vertebra trabecular thickness of pups whose mothers received the calcium-fortified or LCPUFA-supplemented diets. The Prebiotic group had an increase of 11% in the trabecular thickness and a decrease of a 21% in the trabecular separation (FIG. 2).

### Adolescence

Offspring groups, regardless of the diet consumed by the mothers, receive the SPRD diet during the growing period. At the end of this period, offspring body weights are not different among the study groups.

pDEXA examination of the whole body skeleton and the appendicular isolated bones (femur and tibia) do not differ among nutritional intervention groups.

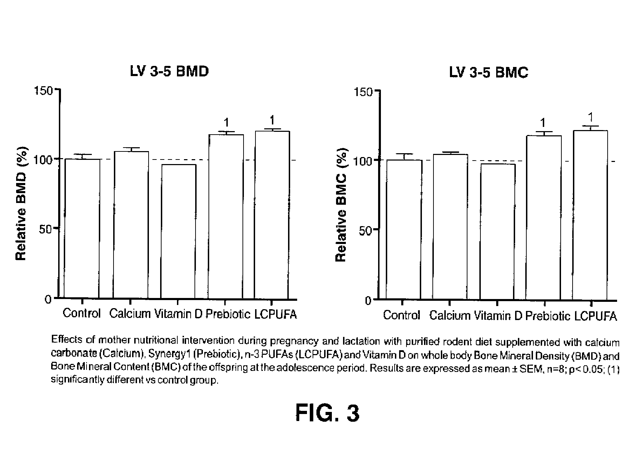

In the offspring whose mother received Prebiotic or LCPUFA-supplemented diet, lumbar vertebra has significantly higher BMD and BMC than in Control group (nearly 20% in the Prebiotic group and greater than 20% for the LCPUFA group in both parameters) (FIG.3). Significantly, no differences are found, with respect to Control group, in offspring whose mother had received the fortified Calcium or Vitamin D-diet for this critical period.

In this Example, only the lumbar vertebra of the adolescent offspring, but not femur or tibia, are examined to determine changes in the bone microarchitecture. The reason is that no effect on BMC or BMD in the appendicular bones is seen.

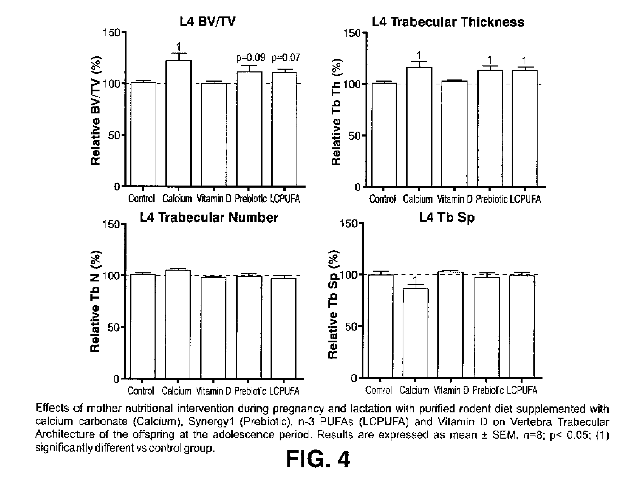

The intervention on the mother, during gestation and lactation periods, with Calcium-fortified, LCPUFA or Prebiotic-supplemented diets, but not with Vitamin D-fortified diet, produces a positive effect on the trabecular architecture of vertebra in their offspring. The two main structural markers relating to cancellous bone quality, BV/TV and trabecular thickness, are enhanced by 22% and 17% in the Calcium group, 11% and 13% in the Prebiotic group and 10% and 13% in the LCPUFA group in comparison with Control group, respectively. The Calcium group also shows a significant decrease on the trabecular separation respective to Control group. (FIG. 4). No effect in either structural parameter is observed in the offspring whose mother received the Vitamin D-fortified diet. The increment in the trabecular thickness is attributable to a positive balance through formation in each remodeling cycle, which results in an increase on the trabecular BMD.

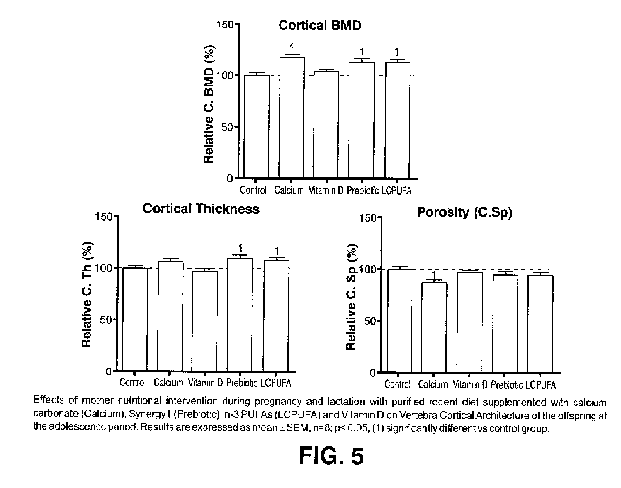

Cortical parameters of the vertebra of the offspring are also positively affected by the LCPUFA or Prebiotic nutritional intervention done in the mothers during the gestational and lactating periods. The supplementation with LCPUFA or Prebiotics produces an increase in the cortical vBMD and cortical thickness and a reduction in cortical porosity. In both groups, cortical vBMD and cortical thickness are enhanced by approximately 13% and 9% with a parallel reduction in the cortical porosity of approximately 6%. Maternal supplementation with calcium also produces an increase in cortical vBMD and a decrease in the cortical porosity, without significantly affecting vertebrae cortical thickness. As well as for the trabecular parameters, Vitamin D group does not show any improvement of the cortical features. (FIG. 5).

### Conclusions From Data Of Example 1

The results discussed above show that at weaning, the LCPUFA group showed significant improvements in BV/TV ratio, trabecular separation, and vertebra trabecular thickness as compared to the Control group. Additionally, at adolescence, the LCPUFA group showed significantly higher BMD and BMC as compared to the Control group, as well as a positive effect on trabecular architecture and cortical parameters. These results indicate that maternal supplementation with LCPUFAs produces positive effects on bone growth and formation as compared to the Control group at weaning and adolescence.

Additionally, the results show at weaning that the Prebiotic group showed a 7% higher BV/TV ratio of the vertebra as compared to the Control group, an 11% increase in trabecular thickness as compared to the Control group, and a 21% decrease in trabecular separation as compared to the Control group. At adolescence, the Prebiotic group showed significantly higher BMD and BMC as compared to the Control group, as well as a positive effect on trabecular architecture and cortical parameters. The results indicate that maternal supplementation with Prebiotics also produces positive effects on bone growth and formation.

The surprising benefits to bone health and architecture in the groups supplemented with Prebiotics or LCPUFAs may point to a higher grade and ossification in the bone of pups whose mothers consumed Prebiotics or LCPUFAs. As such, the Prebiotic or LCPUFA maternal diet supplementation provides a significant bone benefit as compared to the Control group.

### Example 2

In this Example, the effect of DHA or EPA on the differentiation of multipotent mesenchymal stem cells into osteoblasts through regulation of specific osteogenic markers (collagen Type I, osteocalcin, and alkaline phosphatase) is investigated.

Specifically, the efficiency of the LCPUFAs DHA or EPA on the growth, differentiation and function of adult human osteoblasts *in vitro* is evaluated through analyzing mechanisms related to modulation of bone marrow mesenchymal stem cell differentiation into cells of the osteogenic lineage. Human bone marrow cells are grown in either basal or osteogenic media as controls. These control cultures are compared at day 7 and 21 using qPCR technology to cells grown in basal media including either the DHA or EPA at levels of 5, 50, or 100 *µ*M.

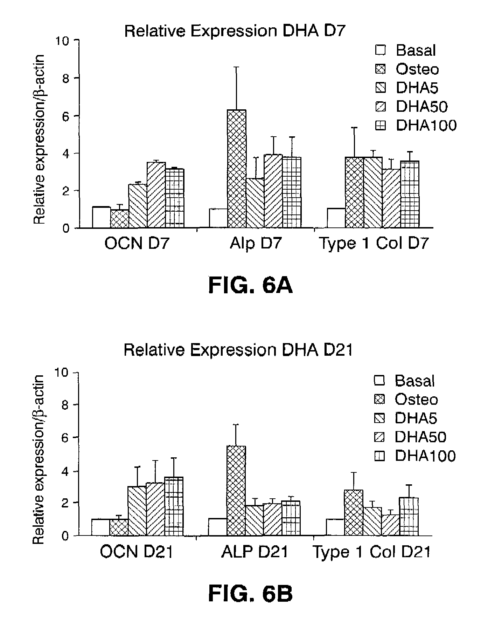

As shown in FIGS. 6A and 6B, on day 7, significantly greater expression of OCN, ALP and Type 1 Collagen is found in cells grown in media supplemented with DHA as compared to those of the basal control cultures. Only cells grown in DHA (5 *µ*M) produce less ALP than the control cells grown in the osteogenic media.

By day 21, ALP expression approaches basal levels, though expression of OCN and Type 1 Collagen is equal to or greater than that of control cells grown in osteogenic media.

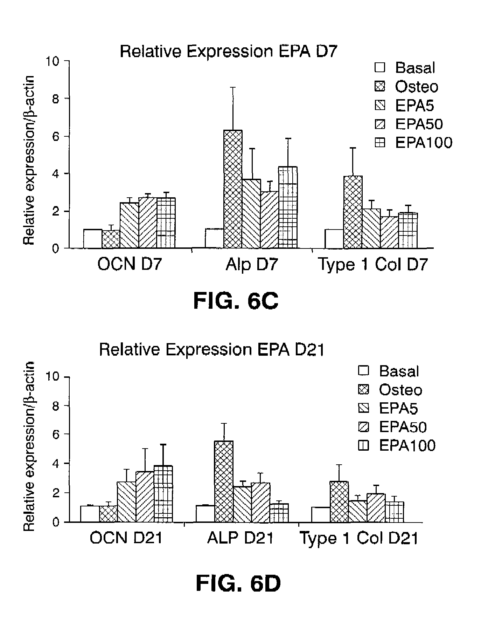

As shown in FIGS. 6A, 6B, 6C and 6D, on day 7, significantly greater expression of OCN, ALP and Type 1 Collagen is found in cells grown in media supplemented with DHA or EPA as compared to control cells grown in basal media. OCN production is also greater than that in osteogenic media, but Type 1 Collagen expression does not achieve equivalence with that from control cells grown in osteogenic media.

By day 21, expression of ALP and Type 1 Collagen has decreased, though OCN expression remains elevated compared to expression in control cells grown in both basal and osteogenic media.

### Conclusions From Data Of Example 2

The results discussed above show that differentiation in osteoinductive culture medium containing the LCPUFA EPA or DHA strongly activated the osteogenic markers. These results point to the osteogenic potential of EPA and DHA in balancing the bone-forming from hMSCs and are significant to the understanding of the effects of these specific LCPUFAs during early bone development to enhance bone mass accretion in an infant from conception through adolescence through maternal supplementation with LCPUFAs.

### Examples 3-7

Examples 3-7 illustrate nutritional powders of the present disclosure, the ingredients of which are listed in the table below. These products are prepared by spray drying methods in separate batches, are reconstituted with water prior to use to the desired target ingredient concentrations. All ingredient amounts are listed as kilogram per 1000 kilogram batch of product, unless otherwise specified.

| **Ingredient** | **Ex. 3** | **Ex. 4** | **Ex. 5** | **Ex. 6** | **Ex. 7** |
|---|---|---|---|---|---|
| Skim Milk Powder | 792.03 | 748.27 | 655.5 | 582.0 | 519.65 |
| Short Chain FOS | 10.0 | 50.0 | 100.0 | 150.0 | 200.0 |
| Extra Fine White Sugar | 81.1 | 81.1 | 81.1 | 81.1 | 81.1 |
| Whole Milk Powder | 44.8 | 44.8 | 44.8 | 44.8 | 44.8 |
| Calcium Phosphate Dibasic | 24.1 | 24.1 | 24.1 | 24.1 | 24.1 |
| Magnesium Phosphate Dibasic | 19.1 | 19.1 | 19.1 | 19.1 | 19.1 |
| Choline Premix | 10.3 | 10.3 | 10.3 | 10.3 | 10.3 |
| Vitamin/Mineral Premix | 8.0 | 8.0 | 8.0 | 8.0 | 8.0 |
| Flavor | 6.0 | 6.0 | 6.0 | 6.0 | 6.0 |
| Powdered DHA (11% (w/w) DHA) | 0.47 | 4.7 | 47.0 | 70.5 | 82.85 |
| Sodium Ascorbate | 3.78 | 3.78 | 3.78 | 3.78 | 3.78 |
| Milk Flavor Powder | 1.5 | 1.5 | 1.5 | 1.5 | 1.5 |

### Examples 8-12

Examples 8-12 illustrate nutritional powders of the present disclosure, the ingredients of which are listed in the table below. These products are prepared by spray drying methods in separate batches, are reconstituted with water prior to use to the desired target ingredient concentrations. All ingredient amounts are listed as kilogram per 1000 kilogram batch of product, unless otherwise specified.

| **Ingredient** | **Ex. 8** | **Ex. 9** | **Ex. 10** | **Ex. 11** | **Ex. 12** |
|---|---|---|---|---|---|
| Skim Milk Powder | 750.8 | 696.085 | 628.84 | 688.51 | 599.75 |
| Short Chain FOS | 50.0 | 105.945 | 90.81 | 110.99 | 200.0 |
| Extra Fine White Sugar | 81.1 | 81.1 | 81.1 | 81.1 | 81.1 |
| Whole Milk Powder | 44.8 | 44.8 | 44.8 | 44.8 | 44.8 |
| Calcium Phosphate Dibasic | 24.1 | 24.1 | 24.1 | 24.1 | 24.1 |
| Magnesium Phosphate Dibasic | 19.1 | 19.1 | 19.1 | 19.1 | 19.1 |
| Choline Premix | 10.3 | 10.3 | 10.3 | 10.3 | 10.3 |
| Vitamin/Mineral Premix | 8.0 | 8.0 | 8.0 | 8.0 | 8.0 |
| Flavor | 6.0 | 6.0 | 6.0 | 6.0 | 6.0 |
| Powdered DHA (11% (w/w) DHA) | 4.7 | 0.47 | 82.85 | 0 | 0 |
| DHA/EPA (10% w/wEPA, 18% w/wDHA) | 0 | 0 | 0 | 3 | 2.75 |
| Sodium Ascorbate | 3.78 | 3.78 | 3.78 | 3.78 | 3.78 |
| Milk Flavor Powder | 1.5 | 1.5 | 1.5 | 1.5 | 1.5 |

## Claims

1. A method of improving bone health in an infant susceptible to developing bone health issues, the method comprising administering to a pregnant woman a nutritional formulation comprising at least one long chain polyunsaturated fatty acid.

2. The method of claim 1 further comprising administering to the woman a nutritional formulation following delivery of the infant and during breastfeeding of the infant, wherein the nutritional formulation comprises an amount of the long chain polyunsaturated fatty acid effective to improve bone health in the infant.

3. The method of claim 1 wherein the nutritional formulation is administered to the pregnant woman daily.

4. The method of claim 2 wherein the nutritional formulation is administered to the woman daily following delivery and during breastfeeding of the infant.

5. The method of claim 1 wherein the long chain polyunsaturated fatty acid is selected from the group consisting of eicosapentaenoic acid, docosahexaenoic acid, and combinations thereof.

6. The method of claim 2 wherein the nutritional formulation is administered to the woman during and after pregnancy to provide an amount of from about 200 mg/day to about 1000 mg/day of docosahexaenoic acid and from about 100 mg/day to about 500 mg/day of eicosapentaenoic acid.

7. The method of claim 6 wherein the woman is administered eicosapentaenoic acid and docosahexaenoic acid in a weight ratio of about 1:2.

8. The method of claim 1 wherein the method further comprises administering to the infant a nutritional formulation comprising at least one long chain polyunsaturated fatty acid from weaning through adolescence.

9. The method of claim 1 wherein the nutritional formulation further comprises at least one prebiotic.

10. A method of increasing bone strength in an infant susceptible to developing bone health issues, the method comprising administering to a pregnant woman a nutritional formulation comprising at least one long chain polyunsaturated fatty acid.

11. The method of claim 10 further comprising administering to the woman a nutritional formulation following delivery of the infant and during breastfeeding of the infant, wherein the nutritional formulation comprises an amount of long chain polyunsaturated fatty acids effective increase bone strength in the infant.

12. The method of claim 10 wherein the nutritional formulation is administered to the pregnant woman daily.

13. The method of claim 11 wherein the nutritional formulation is administered to the woman daily following delivery and during breastfeeding of the infant.

14. The method of claim 10 wherein the long chain polyunsaturated fatty acid is selected from the group consisting of eicosapentaenoic acid, docosahexaenoic acid, and combinations thereof.

15. The method of claim 11 wherein the nutritional formulation is administered to the woman during and after pregnancy to provide an amount of from about 200 mg/day to about 1000 mg/day of docosahexaenoic acid and from about 100 mg/day to about 500 mg/day of eicosapentaenoic acid.

16. The method of claim 15 wherein the woman is administered eicosapentaenoic acid and docosahexaenoic acid in a weight ratio of about 1:2.

17. The method of claim 11 wherein the method further comprises administering to the infant a nutritional formulation comprising at least one long chain polyunsaturated fatty acid from weaning through adolescence.

18. A method of promoting bone formation in an infant susceptible to developing bone health issues, the method comprising:
administering to a pregnant woman a nutritional formulation comprising at least one long chain polyunsaturated fatty acid; and
administering to the woman the nutritional formulation following delivery of the infant and during breastfeeding of the infant, wherein the nutritional formulation comprises an amount of long chain polyunsaturated fatty acids effective to promote bone formation in the infant.

19. The method of claim 18 wherein the long chain polyunsaturated fatty acid is selected from the group consisting of eicosapentaenoic acid, docosahexaenoic acid, and combinations thereof.

20. The method of claim 18 wherein the nutritional formulation is administered to the woman during and after pregnancy to provide an amount of from about 200 mg/day to about 1000 mg/day of docosahexaenoic acid and from about 100 mg/day to about 500 mg/day of eicosapentaenoic acid.
